(19) Europäisches Patentamt
European Patent Office
Office européen des brevets

(11) **EP 1 226 433 B1**

(12) **EUROPÄISCHE PATENTSCHRIFT**

(45) Veröffentlichungstag und Bekanntmachung des Hinweises auf die Patenterteilung:
**07.04.2004 Patentblatt 2004/15**

(51) Int Cl.⁷: **G01N 33/50**, G01N 33/566

(21) Anmeldenummer: **00977466.2**

(86) Internationale Anmeldenummer:
**PCT/EP2000/010806**

(22) Anmeldetag: **02.11.2000**

(87) Internationale Veröffentlichungsnummer:
**WO 2001/033218 (10.05.2001 Gazette 2001/19)**

(54) **VERFAHREN ZUR IDENTIFIZIERUNG VON ANTI-APOPTOTISCH WIRKSAMEN VERBINDUNGEN**

METHOD FOR IDENTIFYING ACTIVE ANTI-APOPTOSIS COMPOUNDS

PROCEDE POUR IDENTIFIER DES COMPOSES INHIBANT L'APOPTOSE

(84) Benannte Vertragsstaaten:
**AT BE CH CY DE DK ES FI FR GB GR IE IT LI LU MC NL PT SE TR**

(30) Priorität: **03.11.1999 DE 19952960**

(43) Veröffentlichungstag der Anmeldung:
**31.07.2002 Patentblatt 2002/31**

(73) Patentinhaber: **CytoTools GmbH**
**64287 Darmstadt (DE)**

(72) Erfinder:
• **FREYBERG, Mark, Andre**
**64287 Darmstadt (DE)**
• **FRIEDL, Peter**
**64823 Gross-Umstadt (DE)**
• **KAISER, Dirk**
**64859 Eppertshausen (DE)**

(74) Vertreter: **Luderschmidt, Schüler & Partner GbR Patentanwälte**
**Industriepark Höchst,**
**Gebäude F 821**
**65926 Frankfurt (DE)**

(56) Entgegenhaltungen:
**EP-A- 0 903 149          WO-A-99/40940**

• **MATEO V ET AL: "CD47 ligation induces caspase-independent cell death in chronic lymphocytic leukemia." NATURE MEDICINE, (1999 NOV) 5 (11) 1277-84. , XP002163073**
• **GAO, A-G. ET AL.: "Thrombospondin modulates alphav/beta3 fucntion through integrin-associated protein." THE JOURNAL OF CELL BIOLOGY, Bd. 135, Nr. 2, Oktober 1996 (1996-10), Seiten 533-544, XP000990435**
• **LORENZO K.K.: 'Apoptosis inducing factor (AIF): a phylogenetically old, capase-independent effector of cell death' CELL DEATH AND DIFFERENTIATION Bd. 6, 1999, Seiten 516 - 524**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Identifizierung von anti-apoptotisch wirksamen Substanzen.

Die Arteriosklerose ("Arterienverkalkung") ist die wichtigste und häufigste krankhafte Veränderung der Arterie. Hierbei kommt es zu einer Veränderung des Gefäßinhalts und zu Läsionen des Endothels (der Endothelzellen) und, dadurch ausgelöst, zu metabolischen und zellulären Reaktionen der Gefäßwand. Arterielle Durchblutungsstörungen sind die häufigste Todesursache in den Industrieländern (ca. 50%). In den meisten Fällen liegt ihnen eine Arteriosklerose zugrunde.

Die Wirksamkeit der gegenwärtig eingesetzten Mittel gegen koronare Herzerkrankungen beruht im wesentlichen darauf, den myokardialen Sauerstoffverbrauch zu senken und der reduzierten Koronardurchblutung anzupassen. Darüberhinaus bewirken diese Mittel eine Dilatation der Koronararterien. Bei arteriosklerotischen Koronarstenosen ist hingegen eine medikamentöse Steigerung der Koronardurchblutung kaum möglich, da die erkrankten Gefäße nicht mehr erweiterungsfähig sind. Alle Medikamente setzen erst in einem sehr späten Stadium der Erkrankung an. Es sind bisher keine Mittel bekannt, die die Ursache der Erkrankung direkt behandeln können oder zur Früherkennung geeignet wären. In Ermangelung einer effektiven Möglichkeit zur Frühdiagnose werden die meisten Patienten erst nach einem Herzinfarkt behandelt. Im fortgeschrittenem Stadium ist meistens nur noch ein operativer Eingriff (z.B. Bypass) möglich.

Die inneren Wände aller Blutgefäße sind von Endothelzellen ausgekleidet. Sie sind an der Regulation verschiedener physiologischer Prozesse, wie beispielsweise der Blutdruckregulation und dem Abbau und der Neubildung der Gefäße, beteiligt. Eine Vielzahl von pathologischen Situationen ist mit der Fehlfunktion von Endothelzellen verbunden, beispielsweise die fokale Entwicklung der Arteriosklerose.

Die Apoptose (Synonym: programmierter Zelltod) ist ein unumkehrbarer Prozeß und ist nicht aufzuhalten. Eine apoptotische Zelle stirbt demzufolge unweigerlich ab.

In der europäischen Patentanmeldung EP-A-0 903 149 wird ein Verfahren zur Identifizierung Apoptose-auslösender Substanzen bei Immunzellen beschrieben. Es wurde gezeigt, daß Substanzen, die an das Integrin-assoziierte Protein (IAP bzw. CD 47) auf der Oberfläche von Immunzellen binden, die Fähigkeit besitzen können, Apoptose auszulösen. Die Wirkmechanismen wurden nicht beschrieben.

Es wurde bereits vorgeschlagen, daß IAP an der Bildung eines spezifischen Calciumkanals beteiligt ist (Schwartz, M. A. et al., The Journal of Biological Chemistry, 268:27, 19931-19934). Ein Rolle dieses hypothetischen Calcium-Kanals bei der Auslösung der Apoptose wurde nicht erwähnt.

An Bifurkationen (Gabelungen) im Gefäßsystem bilden sich häufiger arteriosklerotische Läsionen als in nichtverzweigten Bereichen der Blutgefäße. Im Bereich dieser Läsionen konnten schon apoptotische Endothelzellen beobachtet werden. Eine Beteiligung apoptotischer Endothelzellen an der Entstehung der Arteriosklerose wird diskutiert [Asakura, T., Karino, T., *Circulation Research, 66,* 1045-1066 (1990)].

Mateo et al. (Nature Medicine, Vol. 5, Nov. 1999, p. 1277-1283) enthüllen die Auslösung von Zelltod über Thrombospondin und CD47.

Es sind derzeit keine Mittel bekannt, um das Auftreten der Apoptose in den Endothelien des Gefäßsystems zu verhindern bzw. therapeutisch zu behandeln.

Der vorliegenden Erfindung liegt daher die Aufgabe zugrunde, ein Verfahren zur Verfügung zu stellen, mit dem Substanzen gefunden werden können, die die Apoptose bei Endothelzellen inhibieren. Dieses Verfahren soll einfach in der Handhabung und sicher und kostengünstig in der Durchführung sein. Die so identifizierten Substanzen sollen als Bestandteil von pharmazeutischen Zubereitungen zur Behandlung von Zuständen, bei denen eine Hemmung der Apoptose indiziert ist, insbesondere von Gefäßerkrankungen, besonders bevorzugt der Arteriosklerose, eingesetzt werden.

Gelöst wird diese Aufgabe durch ein Verfahren zur Identifizierung Apoptose-inhibierender Substanzen bzw. antiapoptotisch wirksamer Substanzen, wobei man Zellen kultiviert, die sowohl IAP als auch das Integrin $\alpha_v\beta_3$ exprimieren, und/oder die Zellen zur Bildung einer Apoptose-induzierenden Substanz veranlaßt, und/oder eine Substanz (en) zusetzt, die Apoptose induziert/induzieren, eine Testsubstanz zusetzt und die Apoptoserate mißt.

Überraschenderweise wurde von den Erfindern gezeigt, daß die gleichzeitige Bindung von Thrombospondin-1 (TSP-1) an IAP und Integrin $\alpha_v\beta_3$ bei Endothelzellen Apoptose auslöst. Weiterhin konnte überraschenderweise gezeigt werden, daß das TSP-1 von den Endothelzellen selbst gebildet wird, es sich also um eine selbst-induzierte oder spontane Apoptose handelt. Diese Untersuchungen wurden in herkömmlichen, statischen Zellkulturen durchgeführt. Diese zeichnen sich durch das Fehlen von Strömungen im Zellkulturmedium aus. Es konnte aber unerwarteterweise gezeigt werden, daß von Endothelzellen in einer Perfusionskultur, d.h. unter Bedingungen, bei denen die Zellen mit einem strömenden Zellkulturmedium konfrontiert sind, TSP-1 nicht gebildet wird und Apoptose in dieser Zellkultur nicht oder nur in sehr geringem Maße auftritt.

Eine Supplementation von frischem Medium mit TSP-1 verursacht eine Erhöhung der spontanen Apoptose bei statisch kultivierten Endothelzellen. Diese Erhöhung entspricht in etwa dem Effekt von statisch konditioniertem Medium (d.h. Medium, das vorher schon bei der Kultivierung von HUVEC in statischer Kultur verwendet wurde) (Tabelle 1). Dies zeigt, daß statisch konditioniertes Medium die Fähigkeit besitzt, Apoptose über einen Mediator wie TSP-1 zu induzieren.

Der Begriff "konditioniertes" Medium bedeutet hier ein Zellkulturmedium, das vorher bereits zur Kultivierung anderer Zellen verwendet wurde. Dieses Medium zeichnet sich dadurch aus, daß in ihm lösliche Mediatoren, z.B. Wachstumsfaktoren, Hormone, etc. gelöst sind, welche von Zellen im Laufe ihrer Kultivierung gebildet werden.

Durch den Einsatz eines anti-TSP-1-Antikörpers, welcher an TSP-1 bindet und auf diese Art und Weise neutralisiert, konnte gezeigt werden, daß TSP-1 der Mediator der Apoptose von Endothelzellen ist. Der Effekt von zugegebenem TSP-1 kann ebenso wie der Effekt von statisch konditioniertem Medium durch ein zugesetztes polyklonales Antiserum gegen TSP-1 wie auch durch den Zusatz eines monoklonalen anti-TSP-1-Antikörpers unterdrückt werden (Tabelle 1).

Tab.1

| Kulturbedingungen | Kulturmedium (24 h) | Apoptoserate(%) |
|---|---|---|
| Statisch | Frisches Medium (a) | 0,9±0,1 |
| Statisch | Konditioniertes Medium (b) | 3,0±0,2 |
| Statisch | Konditioniert + anti TSP 1(c) | 0,1±0,1 |
| Statisch | Frisch + TSP 1(e) | 3,0±0,4 |
| Statisch | Frisch + anti TSP 1 (d) | 0,2±0,1 |
| Statisch | Frisch + TSP I+ anti TSP 1(f) | 0,2±0,1 |
| Statisch | Frisch + mAk TSP 1 (g) | 0,4±0,1 |

Die Apoptoserate (%) wurde wie in Beispiel 6 gezeigt bestimmt. Die Buchstaben (a) bis (g) beziehen sich auf die in Beispiel 10 vorgestellten Untersuchungen und die dort gewählten spezifischen Versuchsbedingungen (siehe Beispiel 10).

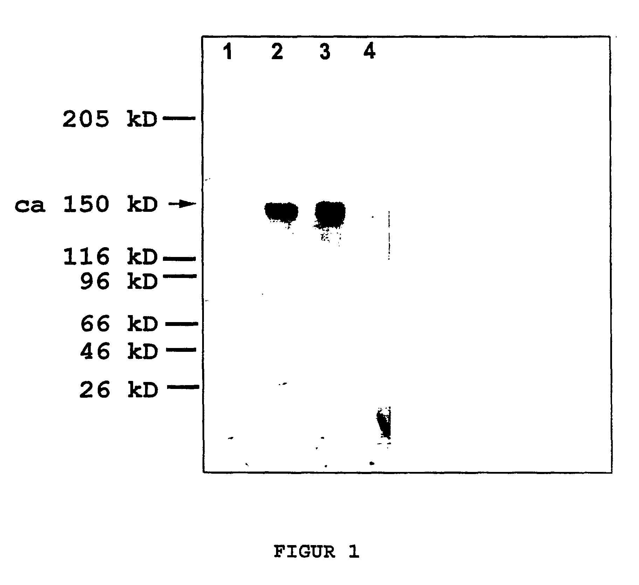

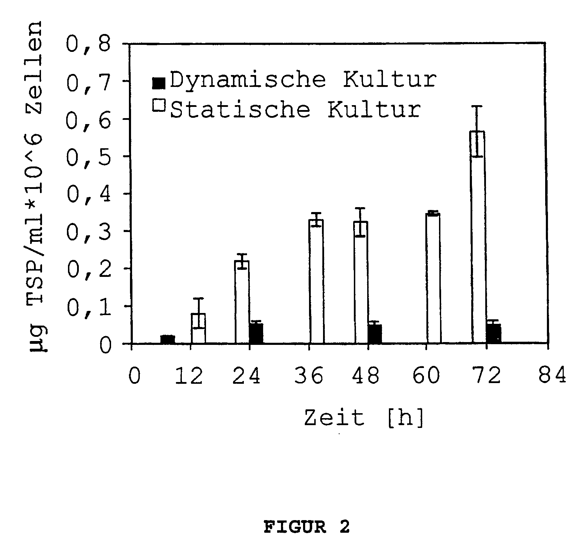

Weiterhin wurde durch Western-Blot-Untersuchungen qualitativ gezeigt, daß TSP-1 nur von statisch kultivierten Endothelzellen sekretiert wird (Figur 1). Die Sekretionsraten wurden für dynamische und statische postkonfluente Kulturen bestimmt (Figur 2). Die Ergebnisse zeigten, daß die Induktion der Apoptose über die Sekretionsrate von TSP-1 kontrolliert werden kann.

Der Begriff "statische Zellkultur(-bedingungen)" bedeutet hier eine Kultivierung von Zellen unter Bedingungen, bei denen stetige, d.h. gleichbleibend gerichtete, laminare Strömungen in dem die Zellen umgebenden Zellkulturmedium nicht auftreten. Zu den "statischen Zellkulturbedingungen" im hier verwendeten Sinne zählen also auch solche Zellkulturbedingungen, unter denen turbulente oder unstetige laminare Strömungen, also z.B. solche mit wechselnden Strömungsrichtungen oder gar mit Strömungsumkehr, auftreten. Der Begriff "dynamische Zellkultur (-bedingungen)" bedeutet hier solche Zellkulturbedingungen, bei denen nur gleichbleibend gerichtete, laminare Strömungsverhältnisse im Zellkulturmedium vorherrschen, d.h. solche Zellkulturbedingungen, wie sie im Stand der Technik z.B. mit Hilfe einer sogenannten Perfusionskultur erreicht werden können. Es ist klar, daß sowohl im Blutgefäßsystem, d.h. in der in-vivo-Situation, als auch unter Zellkulturbedingungen die Verhältnisse der idealisierten physikalischen Strömungslehre nicht erreicht werden. Es handelt sich bei den hier beschriebenen Strömungsverhältnissen also um solche, wie sie näherungsweise allgemein bei Zellkulturexperimenten im Stand der Technik erreicht werden können und verwendet werden.

Je nach Strömungsverhältnissen wirken unterschiedliche Scherkräfte auf die kultivierten Zellen ein. Eine gleichbleibend gerichtete laminare Strömung resultiert dabei in einer Scherkraft, die größer als 0,001 dyn/cm$^2$ ist und deren Vektorsumme größer ist als unter unstetigen Strömungsverhältnissen mit wechselnden Strömungsrichtungen. Statische Zellkultur(-bedingungen) zeichnen sich durch deutlich kleinere (< 0,001 dyn/cm$^2$) bzw. gar keine Krafteinwirkung aus. Dynamische Zellkulturen weisen Scherkräfte von > 0,001 dyn/cm$^2$ bzw. eine Reynoldszahl von > 0,1 auf. Bei Reynoldszahlen von > 200 (-1000) können turbulente Strömungen auftreten (abhängig von der Geometrie der Strömungskammer), die wie statische oder unstetige Strömungsbedingungen keinen protektiven Charakter mehr auf die Auslösung der Apoptose haben.

Die Zugabe von TSP-1 zu einer Perfusionskultur im allgemeinen hat jedoch überraschenderweise keinen Effekt auf die Apoptose. Dies zeigt, daß die Apoptose nicht nur vom Auftreten von TSP-1 im Blutstrom, sondern vielmehr auch vom Auftreten bzw. der Zugänglichkeit spezifischer Rezeptoren auf der Oberfläche der Zellen abhängt. Hierbei konnte überraschenderweise gezeigt werden, daß der Rezeptor Integrin $\alpha_v\beta_3$ auf statisch und dynamisch kultivierten Zellen nachzuweisen ist, hingegen der Rezeptor IAP nur in statischer Kultur in nachweisbaren Mengen exprimiert wird.

Es ist bekannt, daß TSP-1 an das Integrin $\alpha_v\beta_3$ bindet. Die Bindung von TSP-1 an das $\alpha_v\beta_3$-Integrin wird über ein RGD-Sequenzmotiv vermittelt. Daher wurde ein potenter Agonist der RGD-vermittelten Bindung, ein cyclisches Arg-Gly-Asp-(D-Phe)-Val-Peptid (cyclisches RGD-Peptid) (Firma Merck), zu postkonfluenten Endothelzellen gegeben.

Dies hatte jedoch unerwarteterweise keinen Effekt auf die Apoptoserate. Dies zeigte, daß zumindest die alleinige Bindung von TSP-1 an das Integrin $\alpha_v\beta_3$ nicht zur Induktion der Apoptose (Tabelle 2) führt.

Tab.2

| Kulturmedium (48 h) | Apoptoserate (%) |
|---|---|
| Frisches Medium (Kontrolle) | 1,5±0,2 |
| Konditioniertes Medium | 6,4±0,5 |
| Frisch + TSP1 | 6,9±1,2 |
| Frisch + aktives RGD | 1,2±0,5 |
| Frisch + inaktives RGD | 1,0±0,4 |
| Frisch + CBD | 2,0±0,2 |
| Frisch + CBD + aktives RGD | 7,0±0,6 |
| Frisch + CBD + inaktives RGD | 1,7±0,6 |
| Frisch + CBD + aktives RGD + anti TSP1 | 6,3±1,3 |

Die Apoptoserate wurde wie in Beispiel 6 gezeigt bestimmt. Die Kultivierung in frischem Medium dient zur Kontrolle und zeigt die spontane Apoptoserate in statischer Zellkultur. Konditioniertes Medium ist solches, welches bereits 48 bis 72 Stunden mit Endothelzellen inkubiert wurde, so daß es von den Endothelzellen sekretierte Faktoren enthielt. Das konditionierte Medium verursachte eine Apoptoserate in der gleichen Höhe wie frisches Medium + TSP-1 und zeigte dadurch, daß in diesem konditioniertem Medium ein oder mehrere Mediator(en) der Apoptose vorhanden sein mußte(n). Die Herstellung von konditioniertem Medium ist in Beispiel 5 erläutert.

Eine weitere mögliche Interaktion von TSP-1 mit einem Rezeptor auf Endothelzellen ist die Bindung an das IAP über die C-terminale Zellbindungsdomäne (CBD). Die C-terminale Zellbindungsdomäne (CBD) ist eine Domäne des TSP-1, welche spezifisch mit dem IAP weehselwirkt. Ein verkürztes TSP-1, das nur aus dieser C-terminalen Zellbindungsdomäne besteht, wird von der Firma Bachem als CBD-Peptid vertrieben. Die Zugabe des CBD-Peptida führte nicht zu einer Erhöhung der Apoptoserate (Tabelle 2). Die gleichzeitige Zugabe des CBD-Peptids und des cyclischen RGD-Peptids führte jedoch überraschenderweise zu einer deutlichen Erhöhung der Apoptoserate (Tabelle 2), die ähnlich hoch war wie die Erhöhung der Apoptoserate durch die Zugabe von TSP-1. Daraus folgt, daß nur eine gleichzeitige Bindung an IAP und das Integrin $\alpha_v\beta_3$ apoptotisch wirksam ist.

Die Substitution durch ein inaktives RGD-Peptid für eines der beiden anderen Peptide läßt den Effekt wieder verschwinden. Eine simultane Gabe von anti-TSP-1-Antiserum und den beiden aktiven Peptiden hat keinen Effekt auf die Erhöhung der Apoptoserate (Tabelle 2). Dies zeigt, daß die Bildung von TSP-1 während der Inkubation keinen Einfluß hat. Damit konnte überraschenderweise gezeigt werden, daß die Aktivität des TSP-1 zur Induktion der spontanen Apoptose über die gleichzeitige Bindung an IAP und $\alpha_v\beta_3$ vermittelt wird.

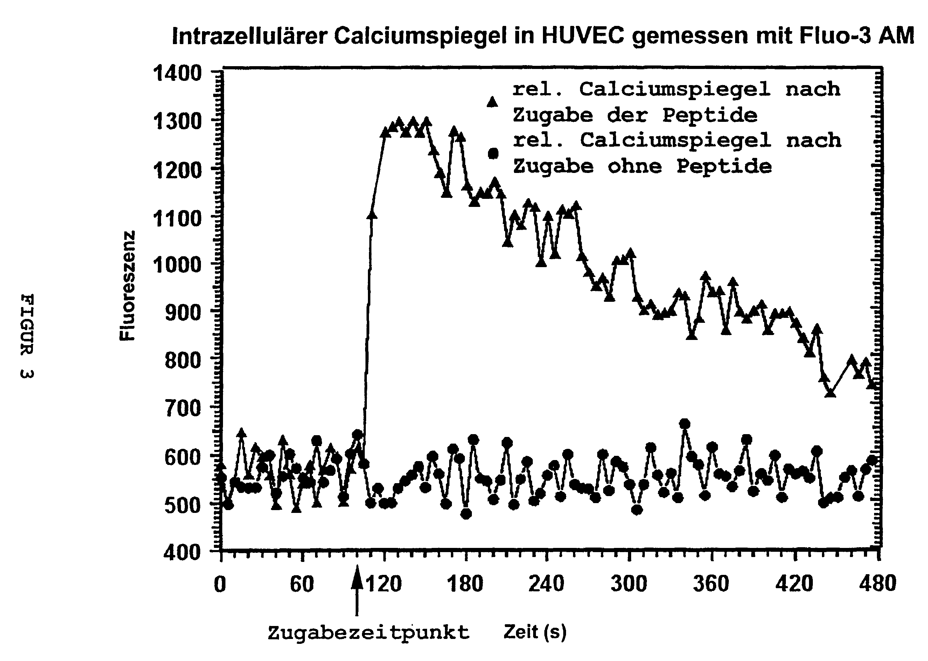

Nach Bindung von TSP-1 an IAP und $\alpha_v\beta_3$ kommt es zu einem Calciumeinstrom in die Zelle (Figur 3). Dieser Calciumeinstrom wird direkt nach Zugabe beobachtet (im Zeitraum von wenigen Sekunden) und ist ein Schritt der Signaltransduktion, die schließlich zur Auslösung der Apoptose führt. Durch den Einsatz von fluoreszierenden Calciumindikatoren [Merrit, J.E. et al., *Biochem. J.* **269**, 513-519 (1990)] kann damit ein sehr schnelles Testverfahren für die Inhibition der Apoptose durch das Ausbleiben des Calciumsignals erstellt werden (Figur 3, näher erläutert in Beispiel 7).

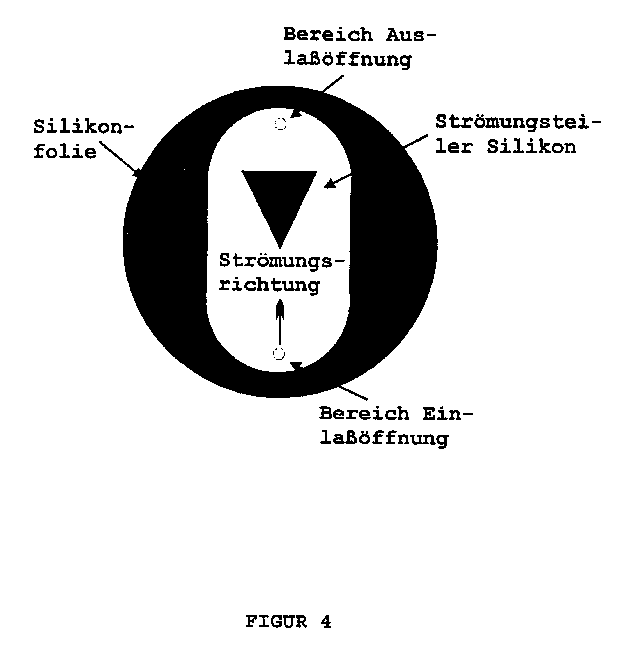

In weiteren von den Erfindern durchgeführten Untersuchungen konnte gezeigt werden, daß nicht nur statische Kulturbedingungen zur Induktion der Apoptose bei entsprechenden Zellen führen, sondern daß unstetige oder turbulente Strömungsverhältnisse hierfür hinreichend sind. Durch das Einbringen eines Hindernisses in eine Perfusionskammer kommt es zu unstetigen Strömungsverläufen. Ein Modell hierfür ist in Figur 4 gezeigt. Eine typische Strömungskammer zeichnet sich durch die Möglichkeit aus, einen Flüssigkeitsstrom zu erzeugen, der mit definierten Flußraten durch eine Strömungskammer geleitet wird. Die Strömung kann z.B. durch eine Pumpe oder die Schwerkraft angetrieben werden. Die jeweilige Flüssigkeit wird über geeignete Schläuche oder Röhren durch die eigentliche Kammer geleitet. Im vorliegenden System handelt es sich hierbei um Silikonschläuche und Edelstahlröhrchen. Die Kammer wird durch eine Petrischale mit einem Polycarbonateinsatz und Silikoneinlagen definiert.

Es gibt auch andere Möglichkeiten, eine laminare Strömung zu erzeugen. Hierfür können auch andere geeignete Apparaturen eingesetzt werden, z.B. eine Plattenkegelapparatur, in der die Strömung durch das Drehen einer flüssigkeitsgefüllten Platte oder das Drehen eines zylindrischen Stempels in der Flüssigkeit der Platte erzeugt wird.

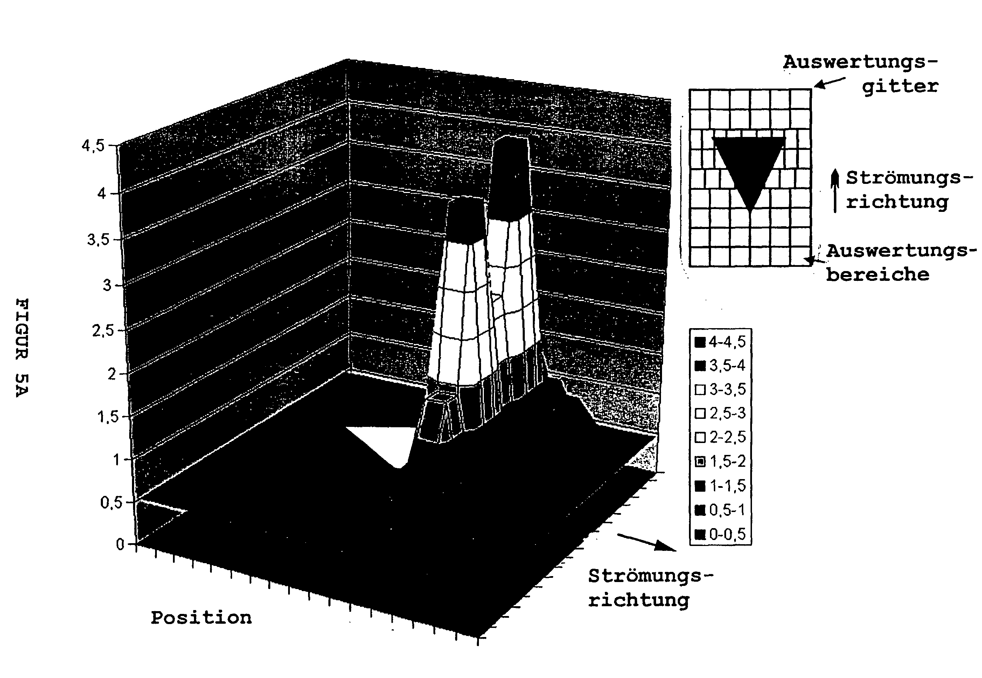

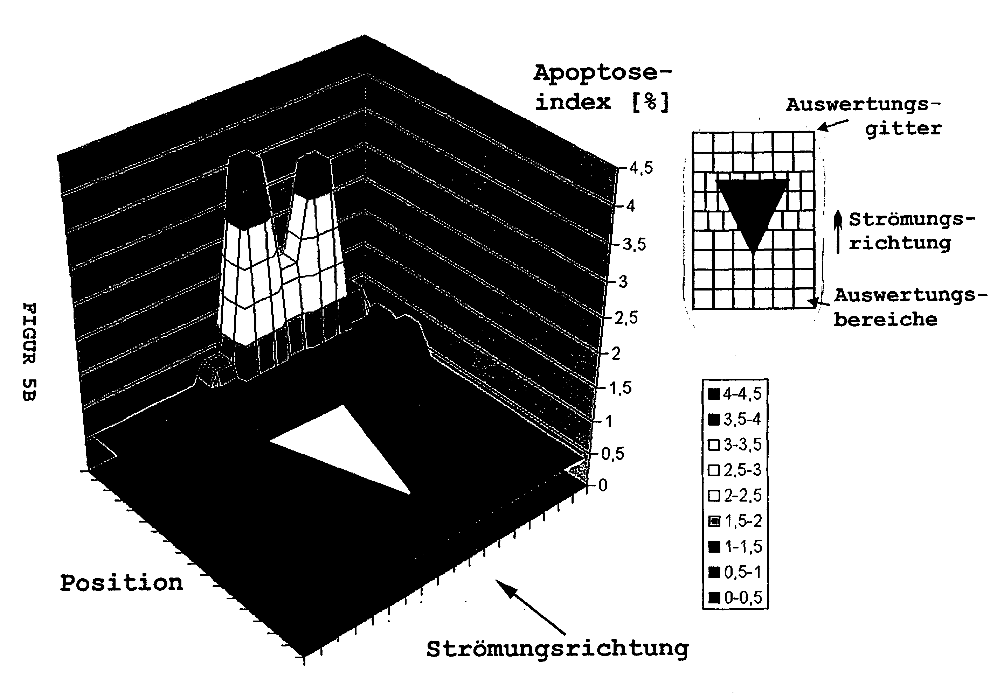

Ferner konnte durch in vitro-Untersuchungen erstmals gezeigt werden, daß es genau in den Regionen unstetiger Strömungsverläufe zur Induktion der Apoptose bei Endothelzellen kommt. Dies konnte durch eine örtlich aufgelöste Untersuchung von apoptotischen Zellen im Perfusionssystem nachgewiesen werden (Figur 5).

Die vorgestellen Ergebnisse zeigen, daß die eigentliche Ursache der Apoptoseinduktion das Ausbleiben einer stetigen laminaren Strömung ist. Dadurch werden die Endothelzellen zur Sekretion von TSP-1 veranlaßt, was anschließend durch die Wechselwirkung mit dem Integrin $\alpha_v\beta_3$ und dem IAP zur Induktion der Apoptose führt.

Aufgrund der vorgestellten Befunde kann ein Testverfahren entwickelt werden, durch das es möglich wird, gezielt nach Inhibitoren der Auslösung von Apoptoseprozessen zu suchen. Damit lassen sich anti-apoptotisch wirksame Substanzen identifizieren. Dazu werden Zellen gezüchtet, die sowohl IAP als auch das Integrin $\alpha_v\beta_3$ exprimieren. Dies können natürliche oder rekombinant erzeugte Zellen sein. Solche Zellen sind einem Fachmann bekannt. Bevorzugt handelt es sich dabei um kultivierte Endothelzellen und in einer besonders bevorzugten Ausführungsform um humane Nabelschnur-Venen-Endothelzellen (HUVEC). Beispiele für geeignete Zellen sind weiterhin Monocyten, Erythrocyten, aktivierte T-Zellen, Fibroblasten, Blutplättchen, CHO-Zellen, glatte Muskelzellen und Ovarientumorzellen. Auch ganze Zellinien können geeignet sein, wie beispielsweise Knochenmarkszellinien. Die in dem erfindungsmäßen Verfahren verwendeten Zellen können auch solche Zellen sein, die so gentechnisch verändert wurden, daß sie rekombinantes, gegebenenfalls unter Beibehaltung der ursprünglichen Bindungseigenschaften verändertes Integrin $\alpha_v\beta_3$ bzw. IAP auf ihrer Zelloberfläche exprimieren.

Diese Zellen werden in geeigneten Zellkulturgefäßen in den geeigneten Zellkulturmedien kultiviert, üblicherweise sind dies Standardmedien, die im Stand der Technik allgemein bekannt sind. Beispielsweise werden die Zellen in DMEM, M-199, IF-Basalmedien usw. kultiviert. Für praktisch alle Zellen und Zellinien sind heute geeignete Kulturmedien vorhanden. Dem Kulturmedium können vor Beginn der Kultur Wachstumsfaktoren und Hormone zugesetzt werden, wie beispielsweise fötales Kälberserum (FCS, fetal calf serum). Die Kultivierung von Säugerzellen erfolgt meist bei 37°C in einer 5%igen $CO_2$-Atmosphäre, die im Zusammenhang mit den in den Zellkulturmedien verwendeten Puffern z.B. Natriumcarbonat-Puffern, eine pH-Wert-Stabilisierung des Zellkulturmediums ermöglicht. Weiterhin können den Zellkulturmedien vor Beginn der Kultivierung Antibiotika zugesetzt werden, die spezifisch mit prokaryotischen kontaminierenden Mikroorganismen wechselwirken und deren Wachstum inhibieren, das Wachstum der eukaryotischen Zellen jedoch weitgehend unbeeinflußt lassen und so die Zellkultur vor Kontamination schützen. Weitere Hinweise und Angaben zur Zellzüchtung können Standardwerken entnommen werden, z.B. Zell- und Gewebekultur, 3. Auflage, Toni Lindl, Jörg Bauer, (1994), Gustav Fischer Verlag.

Geeignete Kulturbedingungen für die Kultivierung von HUVEC und anderen Endothelzellen sind in Beispiel 1 und Beispiel 2 gegeben.

Die Apoptose wird nun durch die Züchtungsbedingungen oder durch Zusatz einer oder mehrerer Apoptoseinduzierender Substanzen induziert. Solche Züchtungsbedingungen sind bevorzugt solche, unter denen regelmäßige laminare Strömungen nicht auftreten, wie vorstehend erläutert.

Zur Induktion bzw. Erhöhung der Apoptoserate können dem Testsystem zusätzlich oder anstelle der gewählten Strömungsbedingungen Mediatoren zugesetzt werden. Der gleichzeitige Zusatz von Mediatoren erhöht die Empfindlichkeit des Testsystems. Die spontane Apoptoserate in einer statischen Zellkultur ist relativ gering, verglichen mit Apoptoseraten, die von Mediatoren, wie TNF-$\alpha$, ausgelöst werden. Bei der spontanen Apoptoserate, ausgelöst durch die statische Zellkulturbedingung, werden Apoptoseraten im Bereich von 0,5% bis 7% der Gesamtzellen erreicht. Bei der durch Mediatoren ausgelösten Apoptose können Apoptoseraten von bis zu 50% erreicht werden.

In dem erfindungsgemäßen Verfahren werden deswegen besonders bevorzugt vor der Messung der Inhibition der Apoptoserate durch potentielle Inhibitoren Substanzen zugesetzt, die eine erhöhte Apoptoserate induzieren, so daß das Meßsignal verstärkt wird. Bevorzugt werden von dem zugegebenen Mediator Apoptoseraten von mindestens 1% bis zu mehr als 10 bis 50% bewirkt. Besonders bevorzugt wird hier TSP-1 dem Zellkultursystem zugesetzt, da es sich bei TSP-1 um den natürlichen Mediator der Apoptose handelt, dessen Wechselwirkung mit den Zellrezeptoren IAP und $\alpha_v\beta_3$ unterdrückt werden soll. Durch Zusatz von TSP-1 können Apoptoseraten von 5%, bevorzugt von 7%, besonders bevorzugt von 10% in Abhängigkeit von der Konzentration des Mediators erreicht werden. Es ist jedoch klar, daß auch andere Substanzen, die in analoger Weise wie TSP-1 mit $\alpha_v\beta_3$ und IAP wechselwirken, erfindungsgemäß geeignet sind. Eine Alternative zu TSP-1 im oben aufgeführten Sinne ist die Steigerung der Apoptoserate durch simultanen Zusatz der Peptide RGD und CBD.

Diese Mediatoren sind Hormone und andere Substanzen, die apoptotisch wirksam sind. Diese Mediatoren werden in gelöster Form der Zellkultur zugegeben, wobei zu beachten ist, daß die verwendeten Lösungen steril sein müssen. Die Sterilität der Lösungen kann auf verschiedene Weisen erreicht werden, vorzugsweise durch Hitzebehandlung (Autoklavieren bei 2 bar und 120°C), oder, wenn dieses Verfahren aufgrund einer besonderen Hitzeempfindlichkeit des Mediators nicht geeignet ist, durch Sterilfiltrieren, beispielsweise mit Nalgene Einmal-Sterilfiltern. Solche Verfahren zur Sterilisierung von Zusätzen für die Zellkultur sind dem Fachmann gut bekannt.

Zur Identifizierung von anti-apoptotisch wirksamen Substanzen werden Testsubstanzen der Zellkultur zugefügt, deren Wirkung auf die Apoptose der kultivierten Zellen untersucht werden soll. Vorzugsweise handelt es sich dabei um monoklonale Antikörper, Antikörperfragmente, polyklonale Antikörper und Peptide. Es können jedoch auch andere Substanzen, von denen vermutet wird, daß sie antiapoptotische Wirkung entfalten können, zugesetzt werden. Diese Substanzen werden vorzugsweise in gelöster Form verabreicht. Die Lösungsmittel müssen hierbei mit der Zellkultur kompatibel sein. Vorzugsweise werden diese Testsubstanzen daher in Pufferlösungen, wie sie allgemein in der Zellkultur weite Verbreitung erlangt haben, gelöst. Beispiele hierfür sind Phosphatpuffer, Natriumcarbonatpuffer und andere. Die gelösten Testsubstanzen werden vorzugsweise vor dem Zusatz in die Zellkultur durch Sterilfiltration (Nalgene Einmal-Sterilfilter) sterilfiltriert (sterilisiert), um kontaminierende Mikroorganismen oder Sporen von Pilzen und ungelöste Bestandteile zu entfernen.

Vorzugsweise wird die gelöste Testsubstanz vor Zusatz zu der Zellkultur temperiert, d.h. der Temperatur der Zellkultur angepaßt. Die Volumina richten sich nach der Konzentration, der eingesetzten Testsubstanz, die ereicht werden soll, und vorzugsweise handelt es sich um geringe Volumina, damit keine Verdünnungseffekte in den Zellkulturmedien auftreten. Die Verfahren, mit denen solche Testsubstanzen in Zellkulturen, vorzugsweise in gelöstem Zustand, eingebracht werden können, sind dem Fachmann gut bekannt.

Die Abnahme der Apoptoserate kann durch jedes geeignete Meßverfahren bestimmt werden. Besonders geeignet als Frühindikator ist die Messung des Ausbleibens des Calciumeinstroms in die Zelle durch Verwendung von intrazellulären Calciumindikatoren. Ein Verfahren, welches geeignet ist, die genauen Apoptoseraten zu bestimmen, ist die Färbung der DNS von apoptotischen Zellen und nachfolgende morphometrische Zellkernanalyse bzw. Analyse des zellulären DNS-Gehalts in einem Durchflußzytometer. Ein Beispiel für einen geeigneten Fluoreszenzfarbstoff stellt DAPI dar. Diese Verfahren sind im Stand der Technik gut bekannt und werden allgemein als Nachweis für apoptotische Zellen verwendet. Nimmt die Anzahl der apoptotischen Zellen nach Einsatz eines potentiellen Inhibitors der Apoptose um mehr als 50%, bevorzugt mehr als 70% und besonders bevorzugt um mehr als 90% ab, bezogen auf die Anzahl apoptotischer Zellen im Testsystem in statischer Kultur, ggf. nach Zusatz einer Apoptose-induzierenden Substanz, so gilt diese Substanz erfindungsgemäß als Inhibitor der Apoptose bei den verwendeten Zellen. Als Frühindikator für Screeningzwecke kann das Ausbleiben des Calciumeinstroms in die Zelle verwendet werden. Substanzen, die zum Ausbleiben des Calciumeinstroms führen, müssen anschließend durch DAPI-Färbung überprüft werden.

Ferner können auch andere DNS-Farbstoffe (z.B. Hoechst 33258) oder gängige Verfahren, wie der TUNEL-Assay (Tdt-mediated X-dUTP nick end labeling; DNS-Brüche), der Nachweis von apoptotischen Enzymen (z.B. PARP, Caspasen) oder Proteinen (z.B. p53, CD95), die Translokation von Phosphatidylserin mit fluoreszierendem Annexin oder der Nachweis der DNS-Leiter im Agarose-Gel eingesetzt werden.

Mit dem erfindungsgemäßen Verfahren können anti-apoptotisch wirksame Substanzen aufgefunden werden. Bevorzugt sind dies Verbindungen, die entweder an Rezeptoren auf der Zelloberfläche, besonders bevorzugt IAP und/oder das Integrin $\alpha_v\beta_3$, oder an humorale Faktoren im Blutkreislauf, besonders bevorzugt TSP-1, so binden, daß die hier beschriebene spezifische gleichzeitige Wechselwirkung zwischen TSP-1 und IAP und $\alpha_v\beta_3$, die zur Induktion der Apoptose führt, nicht stattfindet, so daß die Apoptose nicht induziert werden kann und dadurch ausbleibt.

In einer weiteren bevorzugten Ausführungsform binden diese Substanzen an das IAP und verhindern dadurch den Apoptosespezifischen Calcium-Einstrom in die Zelle, so daß die Apoptose nicht induziert werden kann und somit ausbleibt.

Diese Inhibitoren sind zur Herstellung pharmazeutischer Zubereitungen geeignet, die zur Behandlung von Zuständen verwendet werden können, bei denen eine Unterdrückung der Apoptose erwünscht ist, bevorzugt im Gefäßsystem zur Unterdrückung der Apoptose von Endothelzellen und anderen Zellen (z.B. glatte Muskelzellen). Prinzipiell können mit den durch das erfindungsgemäße Verfahren identifizierbaren anti-apoptotisch wirksamen Substanzen apoptotische Zustände bei allen Zellen inhibiert werden, die IAP und $\alpha_v\beta_3$ auf ihrer Oberfläche exprimieren. Hiermit wird eine Behandlung von Zuständen, bei denen eine Hemmung der Apoptose indiziert ist, wie insbesondere der Arteriosklerose, möglich, die über eine Inhibition des programmierten Zelltodes in den Läsionen zu einer Verbesserung des Gefäßzustandes führt. Auch eine prophylaktische Behandlung wird durch die Verwendung solcher Inhibitoren in pharmazeutischen Zubereitungen möglich.

Aktive mit dem erfindungsgemäßen Verfahren zu testende Wirksubstanzen sind vor allem monoklonale Antikörper und Peptide, die mit gängigen Methoden der Molekularbiologie und der Gentechnologie leicht erhältlich sind. Es ist jedoch klar, daß auch andere Substanzen, die eine entsprechende Wirkung aufweisen, mit dem erfindungsgemäßen Testsystem aufgefunden werden können. Der Begriff "Antikörper" wird hier verwendet zur Beschreibung sowohl von kompletten Antikörpern (d.h. Antikörper, die zwei schwere und zwei leichte Ketten besitzen) als auch von Fragmenten von Antikörpern, die eine Antigenbindestelle besitzen. Die Identifizierung eines anti-apoptotisch wirksamen Anti-TSP-1-Antikörpers ist in Beispiel 10 beschrieben. In ähnlicher Weise können viele andere Antikörper oder Antikörperfragmente getestet werden, um die Induktion der Apoptose zu verhindern.

Es ist klar, daß eine ganze Reihe von Peptiden und Antikörpern (Antikörperfragmente) hergestellt werden können, die in ähnlicher Weise mit dem erfindungsgemäßen Verfahren als anti-apoptotisch wirksam identifiziert werden können. Darüber hinaus können auch andere Substanzen, die nicht Antikörper (Antikörperfragmente) oder Peptide sind, mit Hilfe des erfindungsgemäßen Verfahrens als anti-apoptotisch wirksam identifiziert werden.

Besonders bevorzugte Substanzen, die als Testsubstanzen in dem erfindungsgemäßen Testsystem untersucht werden können, sind niedermolekulare Verbindungen. Solche Verbindungen haben oft keine oder nur geringe Nebenwirkungen, wenn sie als aktives Prinzip in einer pharmazeutischen Zusammensetzung eingesetzt werden. Ein weiterer Vorteil solcher Substanzen ist die Möglichkeit der oralen Verabreichung.

Beispiele hierfür sind cyclische Pentapeptide, wie sie von Haubner et. al., J. Am. Chem. Soc. 1996, 118, 7641-7472, beschrieben wurden. Erfindungsgemäß werden den niedermolekularen Stoffen kleine Peptide, Aminosäuren und Aminosäureanaloga, Steroide, Nukleotide und weitere organisch-chemische Stoffe mit einem Molekulargewicht von $\leq 5000$, bevorzugt $\leq 3000$ und besonders bevorzugt $\leq 2000$ zugerechnet. [Haubner, R., Gratias, R., Diefenbach, B., Goodman, S. L., Jonczyk, A., Kessler, H., *Structural and Functional Aspects of RGD-Containing Cyclic Pentapeptides as Highly Potent and Selective Integrin $\alpha_v\beta_3$ Antagonists*, 118, 7461-7472 (1996)].

Die Herstellung entsprechender Präparate erfolgt nach üblichen Verfahren. Beispielsweise können Peptide oder Antikörper (Antikörperfragmente), die aktiver Bestandteil einer pharmazeutischen Zubereitung sind, in einem pharmazeutisch annehmbaren Träger gelöst werden. Ein Beispiel für einen pharmazeutisch annehmbaren Träger können Pufferlösungen, wie Phosphatpuffer oder Citratpuffer, sein. Zur Aufrechterhaltung der Aktivität der Peptide können auch Reagentien zugesetzt werden, die pharmazeutisch annehmbar sind und beispielsweise ein reduzierendes Milieu in der pharmazeutischen Zubereitung aufrechterhalten.

Abkürzungen

| | |
|---|---|
| IAP: | Integrin-assoziiertes Protein (CD 47) |
| CBD: | C-terminale Zellbindungsdomäne |
| TSP-1: | Thrombospondin 1 |
| RGD: | Arg-Gly-Asp |
| HUVEC: | Humane Nabelschnur-Venen-Endothelzellen |
| HPEC: | Humane Plazentaendothelzellen |
| DAPI: | 4',6-Diamidino-2-phenylindol |

Die folgenden Figuren erläutern die Erfindung näher:

Figur 1 zeigt den Nachweis von Thrombospondin im Überstand von serumfrei kultivierten Endothelzellen (HUVEC). Westernblot-Analyse mit anti-Thrombospondin-Antikörper. Reihe 1: serumfreies Medium; Reihe 2: serumfreies Medium, 4 Tage statisch konditioniert; Reihe 3: 500 ng TSP-1; Reihe 4: serumfreies Medium, 3 Tage dynamisch konditioniert.

Figur 2 zeigt den quantitativen Nachweis des sekretierten TSP-1 im Überstand von statisch und dynamisch kultivierten Endothelzellen (HUVEC).

Figur 3 zeigt die Veränderung des intrazellulären Calciumspiegels von Endothelzellen in statischer Kultur nach dem Einsatz von Thrombospondin bzw. der beiden aktiven Peptide.

Figur 4 zeigt die Geometrie der Strömungskammer und des Strömungsteilers in der Perfusionskammer. Endothelzellen wachsen im freien Bereich.

Figuren 5a und 5b zeigen Auswertungsgitter für Versuche mit dem Strömungsteiler und eine positionsaufgelöste Darstellung des Auftretens der Apoptose in der Perfusionskultur mit unstetigen Strömungsverhältnissen. Nach 2 Tagen Perfusionskultur wird für jeden Bereich die Apoptoserate bestimmt. Hinter dem Strömungsteiler können signifikante Apoptoseraten nachgewiesen werden. Die Apoptoserate hängt von der Position in der Perfusionskammer ab.

Die folgenden Beispiele erläutern die Erfindung näher.

Beispiel 1

Kultivierung humaner Endothelzellen aus Nabelschnur-Venen (HUVEC)

Lösungen (steril) :

- Kulturmedium: IF-Basalmedium + 15% (v/v) NCS, 5 µg/ml Transferrin, 5 µg/ml Heparin, 0,7 µg/ml FGF, 2 mM L-Glutamin [IF-Basalmedium: 1:1-Mischung aus Iscove's Modifiziertem Dulbecco Medium (IMDM) und Ham's F12, beide von Life Technologies, Paisley (England)]
- NCS: Serum neugeborener Kälber (Sebak, Aidenbach)
- FGF: Fibroblastenwachstumsfaktor (Eigene Präparation aus Schweinehirn gereinigt)

Materialien :

- Zellkulturgefäße, gelatiniert

Die Kultivierung von HUVEC erfolgt in gelatinebeschichteten Kulturgefäßen bei 37°C, 5% $CO_2$ und Wasserdampf-gesättigter Luftatmosphäre. Das Kulturmedium wird alle 2-3 Tage gewechselt; bei Konfluenz werden die Zellen mit einer Teilungsrate von 1:3 bis 1:5 passagiert. HUVEC wachsen streng kontaktinhibiert und bilden einschichtige Zellrasen mit der typischen Kopfsteinpflastermorphologie. Bei Konfluenz erreichen die Kulturen Zelldichten von 4-9 x $10^4$ Zellen/$cm^2$. Für Apoptoseuntersuchungen werden ausschließlich HUVEC-Kulturen der Passagen 1-4 verwendet.

Ergänzung

Beschichtung von Kulturgefäßen

Lösungen (steril) :

- Gelatinelösung, 1% (w/v) in Milli-Q-Wasser 1 g Gelatine (zellkulturgetestet) in 100 ml Milli-Q-Wasser suspendieren, durch Autoklavieren für 20 min bei 121°C und 2 bar lösen und bei Raumtemperatur lagern.
- PBS (140 mM NaCl, 3 mM KCl, 8 mM $Na_2HPO_4$, 1,5 mM $KH_2PO_4$)

   - 8 g/l NaCl
   - 0,2 g/l KCl
   - 1,44 g/l $Na_2HPO_4$ x 2 $H_2O$
   - 0,2 g/l $KH_2PO_4$

   Die Salze in einem entsprechenden Volumen Milli-Q-Wasser lösen, 20 min bei 121°C und 2 bar autoklavieren und bei Raumtemperatur lagern. Der pH-Wert wird überprüft und liegt zwischen 7,2 und 7,4.

Materialien :

- Zellkulturgefäße

Durchführung :

Für die Kultivierung adhärent wachsender Zellen werden Kulturgefäße mit Gelatine beschichtet. Der Boden der Zellkulturgefäße wird mit steriler Gelatinelösung bedeckt, die Zellkulturgefäße werden 15 min bei Raumtemperatur belassen. Die Gelatinelösung wird abgesaugt, die Zellkulturgefäße werden einmal mit PBS gewaschen und können so verwendet werden.

Subkultivierung adhärenter Zellen

Lösungen (steril):

- PBS
- Trypsin/EDTA (0,05% (w/v)/0,02% (w/v))

   - 0,1 ml Trypsin-Stammlösung
   - 0,05 ml EDTA-Stammlösung

   Mit sterilem PBS auf 50 ml auffüllen, und in Portionen zu 10 ml bei -20°C lagern.

Materialien:

Zellkulturgefäße, gelatiniert

Durchführung:

Alle aufgeführten Zelltypen werden mit Trypsin/EDTA-Lösung von der Kulturfläche abgelöst. Das Kulturmedium wird abgesaugt, der Boden des Kulturgefäßes kurz mit PBS gewaschen und mit Trypsin/EDTA-Lösung ($\sim$1 ml für eine 25 cm$^2$-Kulturflasche) bedeckt. Die Enzymlösung wird sofort wieder abgesaugt, so daß ein dünner Flüssigkeitsfilm auf den Zellen verbleibt. Die Zellen werden 1-10 min bei Raumtemperatur belassen und das Ablösen der Zellen unter dem Mikroskop verfolgt. Das Ablösen der Zellen kann durch sanftes Aufschlagen des Kulturgefäßes auf der Kante beschleunigt werden. Die Zellen werden in frischem Kulturmedium aufgenommen, eventuell gezählt und in neue Kulturgefäße ausgesät.

Beispiel 2

Kultivierung von Endothelzellen aus Rinderaorta (BAEC)

Lösungen (steril):

- Kulturmedium: IF-Basalmedium + 5% (v/v) NCS, 5 µg/ml Transferrin, 20 µg/ml Heparin, 2 mM L-Glutamin; auf 37°C temperiert

Materialien:

- Zellkulturgefäße, gelatiniert

Durchführung:

Die Kultivierung von BAEC erfolgt in gelatinebeschichteten Kulturgefäßen bei 37°C, 5% $CO_2$ und Wasserdampf-gesättigter Luftatmosphäre. Das Kulturmedium wird alle 2-3 Tage gewechselt; bei Konfluenz werden die Zellen mit einer Teilungsrate von 1:3 bis 1:5 passagiert. BAEC wachsen streng kontaktinhibiert und bilden einschichtige Zellrasen mit der typischen Kopfsteinpflastermorphologie. Die Zelldichte beträgt 7-9 x 10$^4$ Zellen/cm$^2$.

Beispiel 3:

Kultivierung im Perfusionssystem

Lösungen (steril):

- Medium (speziell für die jeweiligen Zellen/HUVEC, siehe Beispiel 1)
- Milli-Q-Wasser (gereinigtes Wasser für Zellkulturen)

Materialien:

- Kleine Petrischale, gelatiniert
- Polycarbonat-Stempel für Petrischalen
- Silikonfolien
- Peek-Verschraubungen
- Silikonschlauch ID 0,5 mm

- Maprene-Pumpenschlauch ID 1 mm
- Aluminiumplatte mit Befestigungen für Stempel
- Greinerröhrchen
- Schott-Flaschen mit Deckeleinsatz aus Teflon

Durchführung:

Die Zellen werden subkultiviert wie beschrieben. Nachdem die adhärenten Zellen Konfluenz erreicht haben, können sie in das Perfusionssystem eingebracht werden. Hierfür wird ein System zusammengebaut. Alle Teile wurden zuvor gründlich mit Milli-Q-Wasser gereinigt. Das komplett zusammengebaute System wird 40 Minuten autoklaviert. Das Vorratsgefäß besteht aus einer Schottflasche mit speziellem Teflon-Deckel. Es sind 3 Löcher im Deckel, durch die Edelstahlröhrchen (3 cm, 1 mm ID) gesteckt sind. Ein Röhrchen ist mit einem Sterilfilter versehen und dient dem Druckausgleich. Die anderen beiden bilden den Ein- und Auslaß für das Medium. Ein Röhrchen ist mit Silikonschlauch nach innen verlängert, damit es in das Medium in der Flasche eintaucht. Von hier geht es weiter über einen Silikon- und Maprene-Pumpenschlauch zu einem 15 ml Greinerröhrchen, das ebenfalls mit einem speziellen Teflondeckel verschlossen ist, in dem wiederum 2 Edelstahlröhrchen stecken. Das Greinerröhrchen dient zur Vorwärmung des Mediums und als Luftblasenfalle. Von hier wird das Medium über einen weiteren Silikonschlauch zum Stempel, bzw. zum Peekverbinder an der Stempeloberseite, weitergeleitet. Der Stempel wird zur Kultivierung auf eine Petrischale mit den betreffenden Zellen gesteckt. Durch einen O-Ring ist der Stempel an der Seite abgedichtet. Damit die Zellen nicht zerdrückt werden, liegt eine speziell zugeschnittene Silikonfolie auf dem Boden der Petrischale. Der Ausgang des Stempels ist wiederum mit Hilfe eines Peek-Adapters an einen Silikonschlauch gekoppelt und führt das Medium zurück ins Mediumreservoir. Vor dem Start des Systems wird das Mediumvorratsgefäß und das Greinerröhrchen mit vorgewärmten Medium befüllt. Das Medium in der Petrischale wird von den Zellen abgesaugt und die Silikonfolien werden vorsichtig eingelegt. Anschließend wird zügig der Stempel aufgedrückt und das System kann an die Peristaltikpumpe angeschlossen werden. Die Pumpgeschwindigkeit kann je nach Bedarf den Zellen angepaßt werden. Sie ist abhängig vom Innendurchmesser des Pumpenschlauchs und der gewählten Drehzahl der Pumpe. Nach einer frei wählbaren Perfusionszeit kann das System in der Sterilbank demontiert und die Zellen für weitere Tests eingesetzt werden. Durch den modularen Aufbau kann das System vielfältig genutzt werden. Mit Hilfe von Ventilen und Schaltern aus der HPLC-Technik ist es möglich, nachträglich Substanzen einzubringen bzw. Medienproben zu entnehmen. Durch den Einsatz von angepaßtem Medium (z.B. IF-Medium mit 25 mM HEPES-Puffer, 12,5 mM NaCl und 0,5 mM Natriumcarbonat) kann auch außerhalb eines Brutschranks gearbeitet werden. Dann wird eine Heizplatte oder eine beheizte Kammer zur Temperierung verwendet.

Für die Versuche mit dem Strömungsteiler wird noch eine weitere Silikonfolie eingelegt (vgl. Figur 4). Hierdurch läßt sich die Strömungscharakteristik in der Kammer verändern.

Beispiel 4

Kultivierung in der Plattenkegelscherungsapparatur

Lösungen:

- Kulturmedium mit 200 E/ml Penicillin, 200 µg/ml Streptomycin
- 70% (v/v) Ethanol

Material:

- Plattenkegelscherungsapparatur, Bussolori et al. (1982) Rev.Sci. Instrum. 53, 1851 - 1854.
- gelatinierte Kulturschale ($\varnothing$ = 94 mm)

Durchführung:

Die Plattenkegelscherungsapparatur wird zunächst mit einem weichen Tuch und 70% Ethanol gereinigt, der Kegel sterilisiert und die Apparatur danach bei 37°C im Wärmeschrank für mindestens 30 min temperiert. Die vorkultivierten Zellen werden mit Kulturmedium gewaschen, mit 0,06 ml/cm$^2$ frischem Kulturmedium versehen und die Kulturschale zügig in die Plattenkegelscherungsapparatur eingebaut.

Der Kegel wird mit Hilfe des Grobtriebes hochgestellt und die Kulturschale samt Deckel in die dafür vorgesehene Fassung sorgfältig eingesetzt. Der Deckel wird abgenommen und der Kegel über der Kulturschale abgesenkt und justiert: die Kegelspitze hat dann einen minimalen Abstand zum Zellrasen und der Kegel läuft rund ohne zu schleifen. Die zusammengebaute Plattenkegelscherungsapparatur wird zur dynamischen Kultivierung der Zellen in den Inkubator bei 37°C, 5% (v/v) $CO_2$ und wasserdampfgesättigter Atmosphäre gestellt.

Beispiel 5

Herstellung von konditioniertem Medium

Lösungen (steril) :

- HUVEC-Medium

Materialien :

- HUVEC, konfluent
- Greinerröhrchen

Durchführung:

HUVEC-Kulturmedium aus Beispiel 1 wird auf einen konfluenten Zellrasen von humanen Nabelschnur-Venen-Endothelzellen gegeben und für 48 Stunden - 72 Stunden konditioniert. Anschließend wird das konditionierte Medium 5 Minuten bei 1000 xg zentrifugiert. Das konditionierte Medium wird bis zum Einsatz bei -20°C eingefroren. Das konditionierte HUVEC-Medium wird für Apoptoseuntersuchungen eingesetzt. Hierfür kann es mit 2 mM Glutamin und 0,7 µg/ml FGF angereichert werden.

Beispiel 6

Bestimmung der Apoptoserate durch Färbung apoptotischer Zellen mit DAPI

DAPI gehört zur Indolfarbstoffgruppe und ist ein selektiver DNS-Farbstoff. Der Farbstoff wird bei 340-360 nm angeregt, und das Emissionsmaximum liegt bei 480 nm. Er wird für Apoptoseuntersuchungen eingesetzt [ vgl. Cohen et *al*., Immunology Today, 14, No. 3, 126-130 (1993)].

Morphologische Auswertung:

Lösungen:

- PBS
- Formaldehydlösung
  4% (v/v) Formaldehyd in PBS
- DAPI-Lösung (Molecular Probes, Leiden, Niederlande)
  2 µg/ml DAPI in Metanol

Materialien:

- Petrischale (35 mm) mit Zellen in Kultur

Durchführung:

Der Kulturüberstand einer Petrischale wird abgesaugt, der Zellrasen wird 15 Minuten mit 1 ml Formaldehydlösung auf Eis fixiert, zweimal mit 2 ml PBS gewaschen, für 15 Minuten mit 0,5 ml DAPI-Lösung versetzt, mit PBS gewaschen und unter dem Fluoreszenzmikroskop ausgewertet. Es wird mit dem UV-Filtersatz und einem 20 x oder 40 x Objektiv gearbeitet. 500-1000 Zellen werden zufällig ausgewählt und die Zellen mit apoptotischen Kernen gezählt.

Der Apoptose-Index wird nach folgender Formel berechnet:

$$\text{Apoptose-Index [\%]} = \frac{\text{Anzahl apoptotischer Zellen}}{\text{Gesamtzellzahl}} \cdot 100$$

Durchflußcytometrie:

Lösungen:

- PBS
- Medium
- Ethanol (p.a.) eisgekühlt (-20°C)
- DAPI-Puffer
- DAPI-Stammlösung
- DAPI-Färbelösung

Durchführung:

Der Kulturüberstand wird abgesaugt, und die Zellen werden, ohne sie mit PBS zu waschen, trypsiniert. Die Zellsuspension wird in Medium aufgenommen, gezählt, bei 800 xg für 5 Minuten zentrifugiert, das Sediment in 0,5 ml IF resuspendiert und in 1,5 ml eiskaltes Ethanol getropft. Die Suspension wird über Nacht bei -20°C gelagert. Nach erneuter Zentrifugation und Resuspendierung des Sediments in 2 ml PBS folgt eine halbstündige Inkubation bei 37°C, eine weitere Zentrifugation, Resuspendierung des Sediments in 5 ml DAPI-Lösung und Auszählung im Durchflußcytometer bei einer Zählrate von 50-300 Ereignissen pro Sekunde. Die erhaltene Auftragung zeigt einen hohen Gipfel an Zellen in der $G_1$-Phase des Zellzyklus, gefolgt von einer Fraktion von Zellen in der S-Phase (mittlere Fluoreszenzintensitäten) und einem letzten Gipfel hoher Fluoreszenzintensitäten, der die Zellen in der $G_2$-Phase repräsentiert. Apoptotische Zellen erscheinen, bedingt durch die abnehmende absolute DNS-M enge pro Zelle, in einem Sub-$G_1$-Gipfel [ Darzynklewicz Z. et al., Cytometry, **13**, 795-808 (1992); Zamai L. et al., Cytometry, **14**, 891-897 (1993)]. Dies zeigt das Auftreten einer Apoptose unter den gewählten Bedingungen.

Beispiel 7

Messung des ausgelösten Calciumeinstroms in die Zellen durch Verwendung des intrazellulären Calciumindikators Fluo-3 AM

Fluo-3 ist ein Calciumindikator, der nach Bindung von $Ca^{2+}$ einen fluoreszierenden Komplex bildet. Der Ester Fluo-3 AM wird durch Diffusion von der Zelle aufgenommen. Erst nach Hydrolyse des Esters in der Zelle entsteht der Calciumindikator Fluo 3. Extrazellulärer Farbstoffester beeinträchtigt also die Messung nicht. Die Messung erfolgt mit normalen Fluorescein-Filtern. Bei einer Anregungswellenlänge von 488 nm (500 nm) liegt das Emissionsmaximum bei 525 nm und erhöht sich durch Calcium Bindung um einen Faktor 100 (200). Der Meßbereich liegt zwischen 0,05 und 20 μM freiem $Ca^{2+}$ [Merritt, J.E. et al., *Biochem. J.* **269**, 513-519 (1990)].

Lösungen:

- PBS

- Medium
- Fluo-3 AM Stammlösung
  50 µg in 10 µl DMSO Dimethylsulfoxid (Molecular Probes, Leiden) (6 mM)
- Fluo-3 AM Einsatzkonzentration
  3 µM in serumfreiem Medium

Materialien:

- Zellen in Kultur

Durchführung:

Konfluente Zellrasen von HUVEC in 24 Loch-Platten werden mit serumfreiem Medium dreimal gespült, für 30 Minuten mit vorgewärmtem serumfreiem Medium mit Fluo-3 AM unter Kulturbedingungen inkubiert, dreimal gespült und die Platte mit einem Fluorimeter gemessen. Zunächst wird die Empfindlichkeit des Geräts justiert und anschließend werden alle 1-10 Sekunden Fluoreszenzwerte aufgenommen, die dem momentanen Calciumspiegel in der Zelle entsprechen. Während die Messung abläuft, können Substanzen zu den Zellen gegeben werden. Hat eine Substanz einen Einfluß auf den Calciumspiegel, kann man das an der Erhöhung bzw. Erniedrigung der Fluoreszenzwerte erkennen (siehe Figur 3). Mit diesem Verfahren ist eine Beurteilung des Calciumspiegels möglich. Der Calciumeinstrom ist ein frühes Anzeichen für eine stattfindende Apoptose.

BEISPIEL 8

Induktion der Apoptose bei kultivierten Endothelzellen mit TSP-1

Die Zellen werden wie in den Beispielen 1 und 2 beschrieben kultiviert. Nach dem Erreichen der vollständigen Konfluenz werden die Zellen für den Test eingesetzt. Zunächst wird Thrombospondin 1 in verschiedenen Konzentrationen (1-600 µg/ml) zu frischem Medium gegeben und mit der Wirkung von konditioniertem Medium auf die spontane Apoptoserate von HUVEC verglichen. Die folgende Tabelle zeigt, daß die Zugabe von Thrombospondin zu einer signifikanten Erhöhung der Apoptoserate führt. Sie entspricht der mit konditioniertem Medium erreichbaren. Die beobachtete Apoptose bei Zugabe von frischem Medium wird durch das während der Dauer des Experiments sekretierte Thrombospondin induziert. Auf perfundierte HUVEC hat Thrombospondin keine Wirkung. Die Apoptoserate wird bestimmt durch Anfärbung apoptotischer Zellen mit DAPI.

Tab. 3

| Kulturbedingungen | Kulturmedium | Apoptoserate (%) nach 24 h |
|---|---|---|
| statisch | frisches Medium | $0,9 \pm 0,1$ |
| statisch | konditioniertes Medium | $3,0 \pm 0,2$ |
| statisch | frisch + TSP-1 (1 µg/ml) | $3,0 \pm 0,4$ |
| dynamisch | frisches Medium | < 0,1 |
| dynamisch | konditioniertes Medium | < 0,1 |
| dynamisch | frisch + TSP-1 (1 µg/ml) | < 0,1 |

Die Apopotoserate wurde nach DAPI-Färbung wie in Beispiel 6 gezeigt angegeben (angegeben sind jeweils der Mittelwert und die Standardabweichung). Die prozentuale Apoptoaerate bezieht sich auf die Gesamtzellzahl.

BEISPIEL 9

Testsystem für anti-apoptotisch wirksame Peptide bzw. Proteine

Die Zellen werden wie in den Beispielen 1 und 2 beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den Test eingesetzt. Es werden Untersuchungen zum Einfluß verschiedener Peptide und Proteine (Tabelle 2) auf die Apoptoserate von Endothelzellen durchgeführt. Hierfür werden die Peptide und Proteine (Tabelle 4) in Kulturmedium (Beispiel 1) gelöst und in den angegebenen Konzentrationen eingesetzt (Tabelle 4). Das Medium mit den gelösten Proben wird zu den Zellen gegeben und für drei Tage unter Kulturbedingungen (Beispiel 1) inkubiert. Anschließend werden die Zellen wie in Beispiel 6 beschrieben zur Bestimmung der Apoptoserate mit DAPI gefärbt. Die Ergebnisse von 3 unabhängigen Experimenten mit der Angabe der Mittelwerte und der Standardabweichung sind in Tabelle 2 zusammengefaßt. Man kann deutlich die Apoptose-induzierende Wirkung von konditioniertem Medium, TSP-1 und von aktivem RGD-Peptid zusammen mit dem CBD-Peptid erkennen.

Als Frühindikator kann die intrazelluläre Calcium-Messung (Beispiel 7) eingesetzt werden. Hierfür werden die Zellen zunächst wie in Beispiel 7 beschrieben vorbehandelt und zum Zeitpunkt der Zugabe der Peptide oder von TSP-1 im Fluorimeter gemessen. Nur TSP-1 oder die gleichzeitige Gabe beider Peptide führt zu einem Anstieg des intrazellulären Calciumspiegels (Figur 3). Die Gabe von frischem Medium oder nur eines Peptids hat keinen Einfluß auf den Calciumspiegel. Das Einströmen von Calcium in die Zellen kann als Frühindikator eingesetzt werden. Eine Verifizierung von positiven Substanzen (Calciumsignal) kann durch die Bestimmung der Apoptoserate mit DAPI (Beispiel 6) nach mindestens 24 Stunden Inkubation erhalten werden. Dadurch können anti-apoptotisch wirksame Verbindungen rasch und eindeutig identifiziert werden.

Tabelle 4

| Ligand | Konzentration | Inkubationszeit | Biologische Aktivität |
|---|---|---|---|
| Thrombospondin | 1 µg/ml | (2-)24-72 h | bindet Integrin $\alpha_v\beta_3$ und IAP |
| RGD-cyclisch, akt. | 250 µg/ml | (2-)24-72 h | bindet Integrin $\alpha_v\beta_3$ |
| RGD-cyclisch, inakt. | 250 µg/ml | (2-)24-72 h | inaktiv |
| IAP-Peptid (CBD) | 400 µg/ml | (2-)24-72 h | bindet IAP |

BEISPIEL 10

Identifikation eines anti-apoptotisch wirksamen Antikörpers mit Hilfe des erfindungsgemäßen Verfahrens

Die Zellen werden wie in den Beispielen 1 und 2 beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den Test eingesetzt. Die Zellen werden mit neuem Medium versorgt; (a) frisches Kulturmedium **[Basisrate der Apoptose]**, (b) konditioniertes Medium (Beispiel 5) **[Apoptose-induzierende Wirkung]**, (c) konditioniertes Medium mit Kaninchen-anti-TSP-1-Antiserum (1:20) **[Apoptose-induzierende und Apoptose-inhibierende Sub stanzen]**, (d) frisches Kulturmedium mit Kaninchen-anti-TSP-1-Antiserum (1:20) **[Apoptose-inhibierende Substanz],** (e) frisches Medium mit 1 µg/ml TSP-1 **[Apoptose-induzierende Sub stanz],** (f) frisches Medium mit 1 µg/ml TSP-1 und Kaninchenanti-TSP-1-Antiserum (1:20) **[Apoptose-induzierende und Apop tose-inhibierende Substanzen],** (g) frisches Medium mit monoklonalem anti-TSP-1-Antikörper (1:30) **[Apoptose-inhibierende Substanz].** Nach 24 h Inkubation unter Kulturbedingungen (Beispiel 1) werden die Zellen fixiert, mit DAPI gefärbt und morphologisch unter dem Fluoreszenzmikroskop untersucht bzw. durchflußcytometrisch untersucht (Beispiel 6). Die apoptotischen Zellen und die Gesamtzellzahl werden bestimmt und der Apoptoseindex berechnet (Prozent apoptotischer Zellen). Die Daten von 3 unabhängigen Experimenten mit der Angabe der Mittelwerte und der Standardabweichung sind in Tabelle 1 angegeben.

Die Versuche mit polyklonalem Antiserum (= Antiserum: polyklonaler Antikörper gegen TSP-1 (aus Kaninchen) ungereinigt; Proteinkonzentration: 900 µg/ml) und mit einem monoklonalen Antikörper gegen TSP-1 (= MAK: monoklonaler Antikörper gegen TSP-1 M 101 ungereinigt; Proteinkonzentration: 300 µg/ml) zeigen eine signifikante Abnahme der Apoptoserate (> 50%) und können als Inhibitoren von natürlichen Apoptose-induzierenden Substanzen (konditioniertes Medium und TSP-1) eingesetzt werden. Sie konnten damit als anti-apoptotisch identifiziert werden. Das Antiserum und der monoklonale Antikörper wurden von Prof. Dr. P. Vischer, Institut für Arterioskleroseforschung Münster, zur Verfügung gestellt.

Das Testsystem kann auch mit dem Calciumindikator Fluo-3 AM durchgeführt werden. Die Zellen werden hierzu ebenfalls wie in den Beispielen 1 und 2 beschrieben kultiviert. Nach dem Erreichen der vollständigen Konfluenz werden die Zellen für den Test eingesetzt. Sie werden mit dem Calciumindikator beladen (Beispiel 7) in ein Fluorimeter gestellt und gemessen. Während der Messung werden die Substanzen b, c, e, f, g zu den Zellen gegeben. In diesen Beispielen ist jeweils ein Aktivator (konditioniertes Medium oder TSP-1) enthalten. In den Beispielen b) und e) kommt es zu einem Einstrom von Calcium und damit zu einer Erhöhung der gemessenen Fluoreszenzwerte. Werden zugleich Apoptose-inhibierende Substanzen zugesetzt, wie in Beispiel c), f) und g), kann kein Anstieg beobachtet werden. Der monoklonale Antikörper gegen TSP-1 oder das polyklonale Antiserum verhindern das Einströmen des Calciums und das Auslösen der Apoptose. Durch die gleichzeitige Gabe eines Aktivators (z.B. TSP-1) und der zu testenden Substanz kann so ein schnelles Testverfahren für potentielle Inhibitoren der Apoptose etabliert werden.

Mit diesem Verfahren wurden zwei weitere Antikörper mit dem beschriebenen Verfahren identifiziert, die als Inhibitoren der Apoptose eingesetzt werden können. Hierbei handelt es sich um:

1. Monoklonaler Antikörper gegen Integrin $\alpha_v$ der Firma Chemicon (Nr. MAB 1960), Klon VNR 139 (100 µl); eingesetzte Verdünnung: 1:100 (mAk/$\alpha_v$)

2. Monoklonaler Antikörper gegen humanes IAP (CD 47) der Firma CYMBUS Biotechnology (Nr. CBL 489), Klon BRIC 126,Konzentration: 100 µg/ml; eingesetzte Verdünnung: 1:100 (1 µg/ml) (mAk/IAP)

Die Antikörper wurden nach Erreichen der Konfluenz mit dem Aktivator TSP-1 (1 µg/ml) in den angegebenen Konzentrationen zu den Zellen gegeben. Nach 48 Stunden Inkubation unter statischen Bedingungen wurden folgende Apoptoseraten gemessen:

| Kulturmedium | Apoptoserate [%] (mit SEM) |
|---|---|
| Frisches Medium + TSP-1 | $7{,}3 \pm 0{,}6$ |
| Frisches Medium + TSP-1 + mAk/a$_v$ | $0{,}9 \pm 0{,}2$ |
| Frisches Medium + TSP-1 + mAk/IAP | $0{,}5 \pm 0{,}1$ |

Tab. 1

| Kulturbedingungen | Kulturmedium (24 h) | Apoptoserate (%) |
|---|---|---|
| Statisch | Frisches Medium (a) | $0{,}9 \pm 0{,}1$ |
| Statisch | Konditioniertes Medium (b) | $3{,}0 \pm 0{,}2$ |
| Statisch | Konditioniert + anti TSP 1(c) | $0{,}1 \pm 0{,}1$ |
| Statisch | Frisch + TSP 1(e) | $3{,}0 \pm 0{,}4$ |
| Statisch | Frisch + anti TSP 1 (d) | $0{,}2 \pm 0{,}1$ |
| Statisch | Frisch + TSP I+ anti TSP 1(f) | $0{,}2 \pm 0{,}1$ |
| Statisch | Frisch + mAk TSP 1 (g) | $0{,}4 \pm 0{,}1$ |

Beispiel 11

Unterdrückung der durch statische Kulturbedingungen ausgelösten Apoptose durch Komplexierung des Calciums oder Inhibierung des Calciumeinstromes

Material (zusätzlich zu Beispiel 1):

- Kuturmedium mit 2,5 mmol/l EGTA

Durchführung:

Die Zellen werden wie in Beispiel 1 beschrieben vorbereitet und nach Erreichen der Konfluenz für weitere 24 Stunden in der Plattenkegelscherungsapparatur (siehe Beispiel 4) kultiviert. Die Kulturschale wird ausgebaut und durch Einlegen eines geeigneten Einsatzes in 12 unabhängige Kulturlöcher (Kulturfläche je Loch 0,8 cm$^2$) unterteilt. Eine Hälfte der Kultur wird in Kulturmedium mit EGTA, die zweite Hälfte in Kulturmedium ohne EGTA kultiviert. Die folgende Tabelle 5 zeigt als Ergebnis, daß die Kultivierung in Kulturmedium mit EGTA zu einer signifikanten Senkung der Apoptoserate führt, d.h. eine Komplexierung des Calcium mit EGTA zu einer Inhibition der Apoptose führt Die Apoptoserate wird durch die Anfärbung der apoptotischen Zellen mit DAPI bestimmt.

Tabelle 5

| Versuchsdauer [Stunden] | Apoptoserate (%) mit EGTA [2,5 mM] | Apoptoserate (%) ohne EGTA |
|---|---|---|
| 0 | 0,40±0,22 | 0,42±0,23 |
| 2 | 0,42±0,22 | 2,22±0,55 |
| 3 | 0,96±0,27 | 2,80±0,64 |
| 4 | 1,56±0,23 | 3,10±0,31 |
| 5 | 2,26±0,35 | 5,33±0,59 |

Die Apoptoserate wurde nach dem Verfahren der DAPI-Färbung wie in Beispiel 6 des Hauptpatents gezeigt ermittelt (angege(angegeben sind jeweils der Mittelwert und die Standardabweichung). Die prozentuale Apoptoserate bezieht sich auf die Gesamtzellzahl.

Beispiel 12

Unterdrückung der durch die biologisch aktiven Peptide ausgelösten Apoptose durch Komplexierung des Calciums

Material (zusätzlich zu Beispiel 4):

- Kulturmedium mit biologisch aktiven Peptiden (vergleiche Hauptpatent Beispiel 10, Tabelle 4)

Durchführung:

Die Zellen werden wie in Beispiel 1 beschrieben vorbereitet und nach Erreichen der vollständigen Konfluenz für weitere 24 Stunden in der Plattenkegelscherungsapparatur (siehe Beispiel 4) kultiviert. Die Kulturschale wird aus der Scherungsapparatur ausgebaut und durch Einlegen eines geeigneten Einsatzes, wie bereits im Beispiel 11 beschrieben in 12 unabhängige Kulturlöcher unterteilt. Die biologisch aktiven Peptide werden zum einen in Kulturmedium mit 0,5 mM EGTA und zum anderen in Kulturmedium ohne EGTA gelöst (Konzentrationen: RGD-Peptid: 250 µg/ml [0,5 mM]; IAP-Peptid: 400 µg/ml [0,5 mM]). Danach wird eine Hälfte der Proben mit Kulturmedium mit EGTA und den aktiven Peptiden und die andere Hälfte mit Kulturmedium, welches zwar die aktiven Peptiden jedoch kein EGTA enthält, versetzt. Das Ergebnis dieser Untersuchungen ist in der nachfolgenden Tabelle 6 dargestellt:

Tabelle 6

| Versuchsdauer [Stunden] | Apoptoserate (%) mit EGTA und mit Peptiden | Apoptoserate (%) ohne EGTA und mit Peptiden |
|---|---|---|
| 0 | 0,45±0,24 | 0,61±0,22 |
| 2 | 1,00±0,29 | 2,08±0,22 |
| 3 | 0,63±0,24 | 2,67±0,18 |
| 4 | 1,25±0,33 | 3,82±0,50 |

Die Zugabe der biologisch aktiven Peptide in einem Kulturmedium ohne EGTA führt über den Versuchszeitraum zu einem deutlichen Anstieg der Apoptose. Durch die Zugabe von EGTA in das Kulturmedium mit den aktiven Peptiden kann eine signifikante Abnahme der Apoptoserate erreicht werden.

Die Apoptoserate wurde nach dem Verfahren der DAPI-Färbung wie in Beispiel 6 gezeigt ermittelt (angegeben sind jeweils der Mittelwert und die Standardabweichung). Die prozentuale Apoptoserate bezieht sich auf die Gesamtzellzahl.

Beispiel 13

Verwendung von Antikörpern oder Peptiden als aktive Wirksubstanz in pharmazeutischer Formulierung

Die in Beispiel 10 identifizierten anti-apoptotisch wirksamen Verbindungen, d.h. der anti-TSP-1-Antikörper, der monoklonale Antikörper VNR 139 und der monoklonale Antikörper BRIC 126, könnten als aktive Wirksubstanzen in pharmazeutischen Formulierungen eingesetzt werden.

Diese Antikörper werden dazu zweckmäßigerweise in einer Konzentration von 3-5 mg pro ml in folgender Formulierung eingesetzt:

- Wasser für Injektionszwecke
- Polysorbat 80
- Dinatriumhydrogenphosphat/Natriumdihydrogenphosphat
- Natriumchlorid

Diese Formulierung wird als Injektionslösung verabreicht.

**Patentansprüche**

1. Verfahren zur Identifizierung anti-apoptotisch wirksamer Substanzen, wobei man

i) Zellen züchtet, die sowohl IAP als auch das Integrin $\alpha_v\beta_3$ exprimieren,
ii) die Zellen zur Bildung von TSP-1 (Thrombospondin-1) veranlasst und/oder TSP-1 oder eine analoge Verbindung mit gleichen Bindungseigenschaften zusetzt,
iii) die Testsubstanz zusetzt, und
iv) die Apoptoserate entweder in an sich bekannter Weise durch Bestimmung des Anteils an Zellen mit apoptotischen Kernen mittels DAPI-Färbung, TUNEL-Assay, DNS-Leiter bzw. Annexin-Färbung oder durch Messung des erhöhten Calcium-Einstroms in die Zelle bestimmt wird.

2. Verfahren nach Anspruch 1, wobei die eingesetzten Zellen Endothelzellen, bevorzugt humane Nabelschnur-Venen-Endothelzellen (HUVEC), humane plazentale Endothelzellen (HPEC), bovine Aortenendothelzellen (BAEC), Schweinehimkapillarendothelzellen (PBMEC), Hybridzellinien (EA.hy 926) und humane mikrovaskuläre Endothelzellen (HMVEC) sind.

3. Verfahren nach Anspruch 1, wobei die eingesetzten Zellen glatte Muskelzellen oder Fibroblasten sind.

4. Verfahren nach Anspruch 1, wobei die eingesetzten Zellen gentechnisch veränderte Zellen, die IAP und $\alpha_v\beta_3$ auf ihrer Oberfläche exprimieren, sind.

5. Verfahren nach Anspruch 2, wobei die Endothelzellen unter Bedingungen kultiviert werden, unter denen gleichbleibend gerichtete laminare Strömungen nicht auftreten.

**Claims**

1. A method for identifying substances with antiapoptotic activity, wherein

i) cells which express both IAP and the integrin $\alpha_v\beta_3$ are cultured,
ii) the cells are caused to produce TSP-1 (thrombospondin-1), and/or TSP-1 or an analogous compound with identical binding properties is being added,
iii) the test substance is added, and
iv) the apoptosis rate is determined in a manner known per se by DAPI staining, TUNEL assay, DNA ladder, annexin staining or by measuring the increased calcium influx into the cell.

2. A method as claimed in claim 1, wherein the cells employed are endothelial cells, preferably human umbilical vein endothelial cells (HUVEC), human placental endothelial cells (HPEC), bovine aorta endothelial cells (BAEC), porcine brain capillary endothelial cells (PBMEC), hybrid cell lines (EA.hy 926) and human microvascular endothelial cells (HMVEC).

3. A method as claimed in claim 1, wherein the cells employed are smooth muscle cells or fibroblasts.

4. A method as claimed in claim 1, wherein the cells employed are genetically modified cells which express IAP and $\alpha_v\beta_3$ on their surface.

**5.** A method as claimed in claim 2, wherein the endothelial cells are cultivated under conditions under which consistently directed laminar flows do not occur.

**Revendications**

**1.** Procédé pour l'identification de substances efficaces anti-apoptotiques, dans lequel on

i) cultive des cellules qui expriment non seulement l'IAP mais aussi l'intégrine $\alpha_v\beta_3$,
ii) incite les cellules à la liaison de la TSP-1 (thrombospondine-1) et/ou on ajoute la TSP-1 ou un composé analogue avec des propriétés de liaison comparables,
iii) ajoute la substance test, et
iv) le taux d'apoptose est déterminé soit d'une façon connue en soi par détermination de la proportion de cellules avec des noyaux apoptotiques au moyen de la coloration au DAPI, du dosage TUNEL, de la migration de l'ADN ou de la coloration de l'annexine soit par mesure de l'augmentation du flux calcique dans les cellules.

**2.** Procédé selon la revendication 1, dans lequel les cellules utilisées sont des cellules endothéliales, de préférence des cellules endothéliales humaines veineuses du cordon ombilical (HUVEC), des cellules endothéliales humaines placentaires (HPEC), des cellules endothéliales aortiques bovines (BAEC), des cellules endothéliales des capillaires du cerveau de porc (PBMEC), des lignées cellulaires hybrides (E.A.hy 926) et des cellules endothéliales humaines microvasculaires.

**3.** Procédé selon la revendication 1, dans lequel les cellules utilisées sont des cellules musculaires lisses ou des fibroblastes.

**4.** Procédé selon la revendication 1, dans lequel les cellules utilisées sont des cellules modifiées par génie génétique qui expriment l'IAP et l'$\alpha_v\beta_3$ sur leur surface.

**5.** Procédé selon la revendication 2, dans lequel les cellules endothéliales sont cultivées dous des conditions qui empêchent des courants laminaires régulièrement parallèles de se produire.

FIGUR 1

**FIGUR 2**

# Intrazellulärer Calciumspiegel in HUVEC gemessen mit Fluo-3 AM

FIGUR 3

EP 1 226 433 B1

Bereich Auslaßöffnung

Silikonfolie

Strömungsteiler Silikon

Strömungsrichtung

Bereich Einlaßöffnung

FIGUR 4

**FIGUR 5A**

Auswertungsgitter

Strömungsrichtung

Auswertungsbereiche

4,5

4

3,5

3

2,5

2

1,5

1

0,5

0

- ■ 4-4,5
- ■ 3,5-4
- □ 3-3,5
- □ 2,5-3
- □ 2-2,5
- ▣ 1,5-2
- ■ 1-1,5
- ■ 0,5-1
- ■ 0-0,5

Position

Strömungsrichtung

FIGUR 5B